# FASCICULE DE BREVET EUROPEEN

(11) **EP 3 727 603 B1**
(45) Date de publication et mention de la délivrance du brevet: **01.03.2023**
(21) Numéro de dépôt: 18826738.9
(22) Date de dépôt: 21.12.2018
(51) Int. Cl.: A61Q 19/08, A61K 8/9789, A61K 8/34, A61K 8/362, A61K 8/60, A61K 36/185

(54) **EXTRAIT DE FLEURS DE KAPOKIER ET COMPOSITIONS COSMETIQUES, PHARMACEUTIQUES OU DERMATOLOGIQUES LE COMPRENANT**
KAPOKBAUMBLÜTENEXTRAKT UND KOSMETISCHE, PHARMAZEUTISCHE ODER DERMATOLOGISCHE ZUSAMMENSETZUNGEN DAMIT
KAPOK TREE FLOWER EXTRACT, AND COSMETIC, PHARMACEUTICAL OR DERMATOLOGICAL COMPOSITIONS CONTAINING SAME

(30) Priorité: 22.12.2017 FR 1763152
(43) Date de publication de la demande: 28.10.2020
(73) Titulaire: Laboratoires Expanscience, 92048 Paris La Défense Cedex (FR)
(72) Inventeur: LECLERE-BIENFAIT, Sophie, 28100 DREUX (FR); BREDIF, Stéphanie, 28210 CROISILLES (FR)
(74) Mandataire: Regimbeau
(86) Numéro de dépôt international: PCT/EP2018/086754
(87) Numéro de publication internationale: WO 2019/122408

(56) Documents cités:
- CN-A- 106 806 161
- CN-A- 107 308 039
- CN-A- 107 468 583
- JP-A- 2003 201 212
- YI-GANG YU ET AL: "In vitro antioxidant activity of Bombax malabaricum flower extracts", PHARMACEUTICAL BIOLOGY, vol. 49, no. 6, 9 mars 2011 (2011-03-09), pages 569-576, XP055495281, NL ISSN: 1388-0209, DOI: 10.3109/13880209.2010.529614
- DATABASE GNPD [Online] MINTEL; 1 mars 2015 (2015-03-01), The Himalaya Drug Company: "Acne-n-Pimple Cream", XP002783360, Database accession no. 3022367
- John Refaat ET AL: "Bombacaceae Between the Ethnomedical Uses and Pharmacological Evidences: A Review", , 1 janvier 2014 (2014-01-01), pages 254-270, XP055495482, Extrait de l'Internet: URL:https://www.ingentaconnect.com/content /ben/npj/2014/00000004/00000004/art00004 [extrait le 2018-07-26]
- JAIN ET AL: "Assessment of credibility of some folk medicinal claims on Bombax ceiba L", INDIAN JOURNAL OF TRADITIONAL KNOWLEDGE, NATIONAL INSTITUTE OF SCIENCE COMMUNICATION AND INFORMATION RESOURCES, NEW DELHI - INDIA, vol. 13, no. 1, 1 janvier 2014 (2014-01-01), pages 87-94, XP018029848,
- JOHN REFAAT ET AL: "Bombacaceae: A phytochemical review", PHARMACEUTICAL BIOLOGY, vol. 51, no. 1, 13 septembre 2012 (2012-09-13), pages 100-130, XP055495271, NL ISSN: 1388-0209, DOI: 10.3109/13880209.2012.698286
- LADEJI O ET AL: "Hypoglycemic properties of aqueous bark extract of Ceiba pentandra in streptozotocin-induced diabetic rats", MEDICINAL & AROMATIC PLANTS ABSTRACTS, SCIENTIFIC PUBLISHERS, SCIENTIFIC PUBLISHERS, NEW DELHI - INDIA, vol. 25, no. 4, 1 August 2003 (2003-08-01) , XP018012600, ISSN: 0250-4367
- Nn: "Ceiba pentandra - Wikipédia", Wikipédia (FR), 13 July 2017 (2017-07-13), pages 1-4, XP055494992, Retrieved from the Internet: URL:https://fr.wikipedia.org/wiki/Ceiba_pe ntandra [retrieved on 2018-07-24]

## Description

L'invention se rapporte à une composition comprenant un extrait de fleurs de de *Bombax costatum.* La composition est avantageusement cosmétique, pharmaceutique ou dermatologique. L'invention a également pour objet un procédé d'extraction, d'un extrait de fleurs de *Bombax costatum* et préférentiellement des calices, ainsi que l'extrait susceptible d'être obtenu par ledit procédé. L'invention concerne également une telle composition ou un tel extrait pour son utilisation dans la prévention ou le traitement des troubles ou pathologies de la peau, des muqueuses ou des phanères, et dans la prévention ou le traitement des troubles vasculaires. L'invention concerne enfin un procédé de soin cosmétique de la peau, des phanères ou des muqueuses, en vue d'améliorer leur état ou leur aspect, consistant à administrer une telle composition ou un tel extrait.

### KAPOKIER

Le Kapokier, en particulier le *Bombax costatum,* appartient à la famille de bombacaceae (APG: Malvaceae). Le *Bombax costatum* est également appelé *Bombax andrieui* ou *Bombax houardii.* Le *Bombax costatum* est plus communément appelé Kapokier à fleurs rouges, fromager, kapokier rouge, faux kapokier, kapokier de forêt, Voaka (en langue Moré), ou Boumbou (en langue Jula).

Bombax est un genre pantropical comprenant 8 espèces : 2 en Afrique, 5 en Asie, et 1 en Océanie, présente jusque dans les îles Salomon. Autrefois, la délimitation du genre Bombax était bien plus large. *Bombax costatum* est parfois considéré comme conspécifique avec *Bombax buonopozense.*

### Usages de Bombax costatum

D'une manière générale, en Afrique, le *Bombax costatum* est un arbre à usage alimentaire assez fréquent : les feuilles sont séchées et mangées comme celles du Baobab ; les fleurs, et notamment les calices, sont également consommées.

Les tubercules des jeunes plants sont consommés par les troupeaux.

Il existe aussi de nombreux usages magico-religieux car il est parfois considéré comme un arbre sacré (Sanou 2013).

Au Burkina Faso, l'écorce est utilisée pour colorer les dents en rouge. On récolte couramment les fleurs pour le calice charnu qui est cuisiné et consommé comme légume. Les feuilles aussi se consomment comme légume. Les fruits immatures et parfois les fleurs sont ajoutés comme épaississants dans les sauces. Les jeunes fruits immatures sont également utilisés dans la préparation d'une boisson. L'huile des graines est consommable. Les fleurs sont très appréciées comme mellifères.

Plusieurs parties de l'arbre sont utilisées en médecine traditionnelle contre différentes maladies. La macération de la racine en poudre est consommée en sauce ou appliquée comme bain contre l'épilepsie. On applique des préparations d'écorce sur les blessures pour favoriser la guérison.

Au Sénégal et en Sierra Leone, des propriétés diurétiques sont attribuées à l'écorce de tige et de racine.

L'écorce est également utilisée pour préparer un médicament contre la trichomonase, l'amibiase et d'autres formes de dysenterie. Un bain dans un extrait d'écorce de tige se prend contre l'aliénation mentale. La poudre d'écorce de tige entre dans la composition d'un médicament appliqué en fumigation contre les maux de tête. Pour traiter les maux de tête ou les maux de dents, une compresse d'écorce peut être fixée sur la tête.

Les feuilles sont prescrites avec d'autres plantes médicinales pour traiter la leucorrhée et la diarrhée. Un extrait de feuilles broyées est absorbé comme boisson contre les problèmes pendant l'accouchement. Un bain dans un extrait de feuilles broyées se prend de manière répétée contre les convulsions. On absorbe une tisane de feuilles séchées ou on l'applique sur le corps contre la rougeole. La décoction de feuilles et d'écorce de tige ou de racine est absorbée comme boisson dans les cas d'oedèmes graves. On boit la décoction de feuilles et de jeunes rameaux pour traiter la jaunisse. La décoction de feuilles est également donnée à boire aux enfants contre le rachitisme.

Différentes parties de la plante sont utilisées pour favoriser la lactation et comme tonique contre la fatigue. On frictionne la peau avec des feuilles mélangées à du beurre de karité contre la lèpre.

Au Mali, la décoction de l'écorce et des feuilles et de parties d'autres plantes se prend contre les troubles de la menstruation. Les feuilles sont émollientes et un bain chaud dans une décoction de feuilles peut être prescrit aux patients fiévreux, en particulier les enfants. Les feuilles entrent également dans les traitements de l'ankylostome et les fleurs dans ceux contre le ténia.

Au Niger, on a trouvé que le fruit contient 20-25% de protéines. Les graines ont une forte teneur en huile (20%). La composition en acides gras de l'huile est la suivante : acide caproïque 3%, acide caprylique 7%, acide palmitique 8%, acide stéarique 3%, acide oléique 49%, acide linoléique 13%, acide rachidique 3%, acide lignocérique 1-2%, et autres 13%. L'extrait mucilagineux des fleurs est principalement constitué de sucres, rhamnose et arabinose ; il constitue un adhésif approprié pour faire des panneaux de particules.

Toutes ces données ont été tirées du rapport ethnobotanique rédigé par le Dr. Lassina SANOU, Colonel des Eaux et Forêts, il travaille pour le Ministère de l'Environnement et du Développement Durable au Centre National des Semences Forestières (CNRS) du Burkina Faso, basé à Ouagadougou (décembre 2014).

CN107308039 et CN106806161 divulguent des produits cosmétiques contenant des extraits des fleurs de Bombax sp.

### OPC

Les Oligomères ProanthoCyanidiques, plus connus sous le nom d'OPC ou de procyanidines, sont des composés phénoliques présents dans de nombreux végétaux. Les OPC sont des polymères de flavonoïdes, précurseurs des tanins. Les monomères constitutifs des procyanidines sont la catéchine et l'épicatéchine. Les procyanidines sont donc constitués d'un même motif, appelé catéchine (formules Ia et Ib) ou épicatéchine (formule IIa et IIb), répété 2, 3, 4, ou 5 fois :

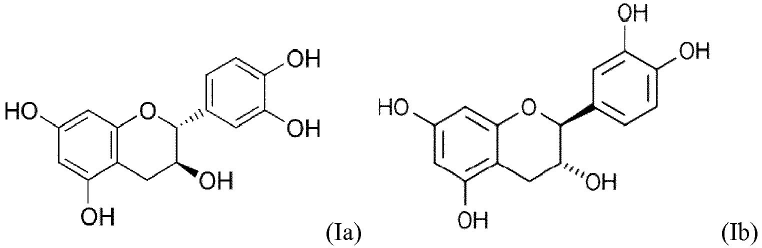

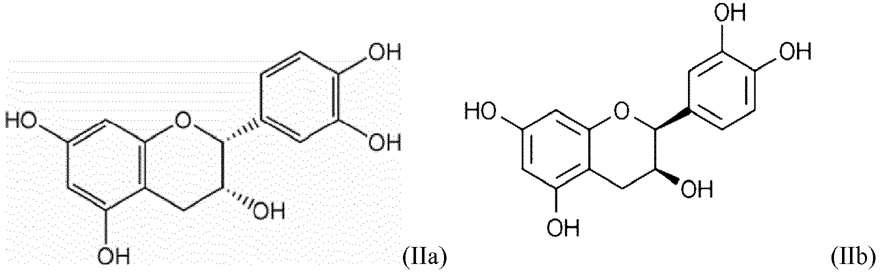

Au-delà de 6 répétitions, on appelle les molécules des polymères ProanthoCyanidiques, de poids moléculaire plus importants. Ils sont très faiblement absorbés et ne présentent, par conséquent, qu'une très faible activité biologique.

Les OPC sont connus pour leurs propriétés en tant que angio-protecteurs, inhibiteurs de l'enzyme de conversion de l'angiotensine, de l'élastase et de la trypsine, et protecteurs des parois vasculaires par liaison au collagène du tissu conjonctif et inhibition des collagénases, améliorant ainsi l'intégrité, la résistance et l'élasticité des parois, ainsi que pour leurs propriétés antioxydante, anti-radicaux libres, anti-inflammatoires et antivirale.

### DESCRIPTION DE L'INVENTION

La Demanderesse a découvert que les extraits de fleurs, de préférence de calices, de *Bombax costatum,* présentent des propriétés cosmétiques et dermatologiques jamais décrites jusqu'à présent. En particulier, c'est la première fois que de tels extraits de fleurs, notamment de calices, de *Bombax costatum,* sont utilisés en tant que tels, pour leurs propriétés spécifiques.

L'invention a pour objet un extrait riche en polyphénols de fleurs, de préférence de calices, de *Bombax costatum,* tel que défini dans la revendication 1.

En botanique, la fleur est la partie de la plante comprenant, de l'extérieur vers l'intérieur, lorsqu'elle est complète :
- le calice, formé par l'ensemble des sépales ;
- la corolle, formée par l'ensemble des pétales ;
- l'androcée, c'est-à-dire l'ensemble des étamines (partie mâle), qui produit le pollen ; et
- le gynécée ou pistil, formé par l'ensemble des carpelles (partie femelle).

Dans la cadre de la présente invention, la « fleur » comprend au moins la corolle et le calice. La fleur peut également être complète.

L'extrait selon l'invention est de préférence un extrait de calices de *Bombax costatum.*

Par « extrait riche en polyphénols », on entend un extrait comprenant majoritairement ou essentiellement des polyphénols.

L'extrait selon l'invention comprend ainsi au moins 15% en poids de polyphénols, plus avantageusement au moins 20% en poids, par rapport au poids total de l'extrait sec ; soit au moins 2,5 mg de polyphénols par ml d'extrait liquide (en équivalent acide gallique). Les pourcentages sont exprimés par rapport au poids total dudit extrait sec (avant ajout éventuel d'un support de séchage). Avantageusement, l'extrait selon l'invention comprend de 15% à 40 %, plus avantageusement de 20% à 40%, en poids de polyphénols, par rapport au poids total de l'extrait sec. L'extrait selon l'invention comprend en outre au moins 15 % en poids d'OPC, exprimés en équivalent catéchine, par rapport au poids total dudit extrait sec, c'est-à-dire au moins 1,9 mg d'OPC par ml d'extrait liquide. Avantageusement, l'extrait selon l'invention comprend de 15% à 25%, en poids d'OPC, exprimés en équivalent catéchine, par rapport au poids total dudit extrait sec.

Avantageusement, l'extrait selon l'invention comprend en outre au moins 6 %, avantageusement de 6% à 10%, en poids d'acides organiques, en particulier d'alpha-hydroxy acides (AHA), notamment d'acide acétique, malique, d'acide lactique, d'acide citrique ou leurs mélanges, par rapport au poids total de l'extrait sec.

Avantageusement, l'extrait selon l'invention comprend en outre au moins 5 %, avantageusement de 5% à 10%, en poids de flavonoïdes, exprimés en équivalent rutine, par rapport au poids total dudit extrait sec.

Selon un aspect avantageux, l'extrait selon l'invention comprend principalement (classés par ordre décroissant en fonction de leur teneur dans l'extrait) : des polyphénols, des OPC, des acides organiques et des flavonoïdes.

En particulier, un tel extrait contient avantageusement (% molécules sur extrait sec obtenu et par ordre décroissant) :
- Environ 20% de polyphénols - en équivalent acide gallique
- Environ 15% d'OPC (Oligomères ProanthoCyanidiques - en équivalent catéchine),
- Environ 6% d'acides organiques (acides citrique, malique majoritairement)
- Environ 4% de flavonoïdes (en équivalent rutine).

Dans le cadre de la présente invention, l'extrait riche en polyphénols décrit ci-dessus est avantageusement obtenu par extraction solide/liquide des fleurs, de préférence des calices, de *Bombax costatum,* dans un solvant choisi parmi l'eau, l'éthanol, les glycols, et leurs mélanges. Le solvant d'extraction est plus particulièrement choisi parmi les mélanges binaires eau/glycols, avantageusement dans une proportion comprise entre 30 et 90%, typiquement entre 50 et 90%, de glycol. En particulier, le solvant d'extraction est choisi parmi les mélanges binaires eau/propanediol ou eau/propylène glycol, en particulier eau/propanediol, plus particulièrement eau/1,3-propanediol.

Plus avantageusement, l'extrait riche en polyphénols décrit ci-dessus est obtenu par extraction solide/liquide des fleurs, de préférence des calices, de *Bombax costatum,* dans un solvant choisi parmi l'eau, les glycols, et leurs mélanges. Le solvant d'extraction est plus particulièrement choisi parmi les mélanges binaires eau/glycols, avantageusement dans une proportion comprise entre 30 et 90%, typiquement entre 50 et 90%, de glycol dans l'eau. En particulier, le solvant d'extraction est choisi parmi les mélanges binaires eau/propanediol et eau/propylène glycol, en particulier eau/propanediol, plus particulièrement eau/1,3-propanediol.

Avantageusement, les glycols cités ci-dessus sont des glycols d'origine végétale, en particulier le propanediol et/ou le propylène glycol, plus particulièrement le proponediol, notamment le 1,3-propanediol.

De manière plus avantageuse, l'extrait riche en polyphénols décrit ci-dessus est obtenu par extraction solide/liquide des fleurs, de préférence des calices, *Bombax costatum,* dans un mélange binaire eau/1,3-propanediol, avantageusement dans une proportion comprise entre 30 et 90%, typiquement entre 50 et 90%, de 1,3-propanediol dans l'eau. Ce solvant particulier permet d'obtenir de meilleurs rendements en actifs visés, notamment en polyphénols, OPC, flavonoïdes et/ou acides organiques.

De manière avantageuse, lorsque l'extraction solide/liquide des fleurs, de préférence des calices, de *Bombax costatum,* est effectuée dans un mélange binaire eau/solvant, les proportions de solvant dans l'eau sont comprises entre 50 et 90%.

L'invention a également pour objet un procédé de préparation d'un extrait riche en polyphénols de fleurs, de préférence de calices, de *Bombax costatum,* avantageusement de calices de *Bombax costatum,* comprenant au moins une étape d'extraction solide/liquide dans un solvant ou un mélange de solvant approprié et dans les conditions optimales de pH, durée et de température, connues de l'homme de l'art.

Avantageusement selon l'invention, le procédé de préparation d'un extrait riche en polyphénols de fleurs, de préférence de calices de *Bombax costatum,* comprend les étapes successives suivantes :
a) broyage des fleurs, notamment des calices ;
b) extraction des fleurs, notamment des calices, broyés dans un solvant ou un mélange de solvant approprié dans des conditions optimales de durée, pH et température ;
c) séparation de la phase solide et de la phase liquide par décantation, et/ou centrifugation et/ou filtration successive; et
d) Eventuellement, séchage de l'extrait obtenu à l'étape c).

L'étape a) de broyage de la plante peut être réalisée par des méthodes connues de l'homme du métier, notamment à l'aide d'un broyeur à couteaux, d'un broyeur à marteaux, etc.

A l'étape b), la phase d'extraction est réalisée de préférence en présence d'eau et/ou de glycols seul ou en mélange binaire. Avantageusement, le solvant d'extraction utilisé à l'étape b) est choisi parmi les mélanges binaires eau/glycols, avantageusement dans une proportion comprise entre 30 et 90%, typiquement entre 50 et 90%, de glycol dans l'eau. En particulier, le solvant d'extraction est choisi parmi les mélanges binaires eau/ propanediol ou eau/propylène glycol, en particulier eau/propanediol, plus particulièrement eau/1,3-propanediol. Plus avantageusement, le solvant d'extraction utilisé à l'étape b) est choisi parmi les mélanges binaires eau/glycols, avantageusement dans une proportion comprise entre 30 et 90%, typiquement entre 50 et 90%, de glycol dans l'eau. En particulier, le solvant d'extraction est choisi parmi les mélanges binaires eau/propanediol et eau/propylène glycol, en particulier eau/propanediol, plus particulièrement eau/1,3-propanediol.

Avantageusement, les glycols cités ci-dessus sont des glycols d'origine végétal, en particulier le propanediol et/ou le propylène glycol, plus particulièrement le propanediol, notamment le 1,3-propanediol.

De manière plus avantageuse, le solvant d'extraction utilisé à l'étape b) est un mélange binaire eau/1,3-propanediol, avantageusement dans une proportion comprise entre 30 et 90%, typiquement entre 50 et 90%, de 1,3-propanediol dans l'eau. Ce solvant particulier permet d'obtenir de meilleurs rendements en actifs visés, notamment en polyphénols, OPC, flavonoïdes et/ou acides organiques.

De manière avantageuse, lorsque l'extraction solide/liquide des fleurs, de préférence des calices, de *Bombax costatum,* est effectuée dans un mélange binaire eau/solvant, les proportions de solvant dans l'eau sont comprises entre 50 et 90%.

En outre, l'étape b) d'extraction solide/liquide est réalisée de préférence à une température comprise entre 20°C et 90°C, en particulier entre 30°C et 80 °C, plus particulièrement entre 30°C et 75°C, typiquement 50°C.

La durée d'extraction est avantageusement comprise entre 30 minutes et 4h, en particulier entre 1h et 3h, avantageusement elle est d'environ 2 heures.

L'étape c) de séparation de la phase solide et de la phase liquide est réalisée par des méthodes connues de l'homme du métier, notamment par décantation, centrifugation et/ou filtrations successives, de préférence par filtration. L'étape c) est effectuée jusqu'à parfaite limpidité et propreté microbiologique.

Avantageusement, l'extrait riche en polyphénols selon l'invention peut être stabilisé par l'étape de séchage d), par des procédés connus de l'homme de l'art.

L'étape d) de séchage peut, par exemple, être réalisée en présence d'un support de type, par exemple, maltodextrines ou fibres d'acacia (Fibregum^{®} société CNI). La teneur en support varie typiquement selon un ratio allant de 0% à 80% de support par rapport au pourcentage de matière sèche obtenu dans la forme liquide de l'extrait.

L'extrait est préférentiellement séché par lyophilisation ou atomisation afin d'obtenir une poudre finale. La poudre finale comprend avantageusement 30 à 70% en poids de matière sèche de l'extrait, le complément à 100% étant le support de lyophilisation ou d'atomisation. Plus avantageusement la poudre finale comprend 50% de matière sèche issue de l'extrait et 50% de support de lyophilisation ou d'atomisation.

Selon un autre mode de réalisation avantageux, l'extrait selon l'invention peut être stabilisé physiquement et microbiologiquement par un solvant choisi parmi les glycols, tels que le propanediol, typiquement le 1,3-propanediol, ledit solvant étant présent dans une proportion adaptée à une telle stabilisation. De préférence, le glycol, en particulier le propanediol, préférablement le 1,3-propanediol, sera présent seul ou en combinaison avec l'eau dans une proportion comprise entre 30 et 90% et préférentiellement entre 50 et 90% de glycol dans l'eau.

Ainsi, la présente invention porte en outre sur une composition comprenant un extrait riche en polyphénols de fleurs, de préférence de calices, de *Bombax costatum,* selon l'invention, un solvant choisi parmi les glycols, tels que le propanediol, en particulier le 1,3-propanediol, en quantité efficace pour une action de stabilisation physique et microbiologique, et éventuellement de l'eau. Les quantités efficaces pour une action de stabilisation physique et microbiologique sont avantageusement telles que décrites ci-dessus.

Préférentiellement, à titre d'exemple, l'extrait riche en polyphénols selon l'invention peut être obtenu selon le procédé suivant :
a') mise en solution de la fleur broyée à 5% (p/p) dans un mélange 1,3- propanediol / eau (60/40) ;
b') extraction sous agitation pendant 2h à 40°C ;
c') Purification par étapes de filtrations successives ; et
d') Filtration stérile.

Dans la suite de la description, on parlera d'« extrait selon l'invention » pour désigner l'extrait en tant que tel, tel que défini ci-dessus, ou l'extrait susceptible d'être obtenu par le procédé selon l'invention tel que décrit ci-dessus. L'extrait susceptible d'être obtenu par le procédé selon l'invention tel que décrit ci-dessus présente la même composition que l'extrait selon l'invention en tant que tel, tel que défini ci-dessus.

L'invention a également pour objet une composition comprenant un extrait riche en polyphénols de fleurs, de préférence de calices, de *Bombax costatum,* notamment de calices de *Bombax costatum,* selon l'invention, en tant que principe actif, et le cas échéant un excipient approprié. L'extrait selon l'invention est tel que défini dans les paragraphes ci-dessus concernant l'extrait en tant que tel et ceux concernant l'extrait susceptible d'être obtenu par le procédé selon l'invention.

La composition est avantageusement cosmétique, pharmaceutique ou dermatologique. Ladite composition est de préférence formulée pour être administrée par voie topique externe.

Avantageusement, la composition selon l'invention comprend de 0,001 à 10 %, typiquement de 0,01 à 5 %, en poids d'extrait selon l'invention, le poids de l'extrait étant exprimé en extrait sec, par rapport au poids total de la composition.

La composition selon l'invention peut comprendre en outre un ou plusieurs autres principes actifs.

La composition selon l'invention peut être formulée sous la forme de différentes préparations adaptées à une administration topique et incluent notamment les crèmes, les émulsions, les laits, les pommades, les lotions, les huiles, les solutions aqueuses ou hydroalcooliques ou glycoliques, les poudres, les patchs, les sprays, les shampooings, les vernis ou tout autre produit pour application externe.

Selon sa nature (cosmétique, pharmaceutique ou dermatologique), la composition selon l'invention peut en outre comprendre au moins un excipient cosmétiquement, pharmaceutiquement ou dermatologiquement acceptable. Notamment, la composition selon la présente invention peut en outre comprendre au moins un adjuvant cosmétiquement, pharmaceutiquement ou dermatologiquement connu de l'homme du métier, choisi parmi les tensioactifs, les épaississants, les conservateurs, les parfums, les colorants, des filtres chimiques ou minéraux, les agents hydratants, les eaux thermales, etc. L'homme du métier sait adapter la formulation de la composition selon l'invention en utilisant ses connaissances générales.

Les posologies et les formes galéniques optimales des compositions selon l'invention peuvent être déterminées selon les critères généralement pris en compte dans l'établissement d'un traitement pharmacologique, dermatologique ou cosmétique adapté à un patient ou à un animal, comme par exemple l'âge ou le poids corporel du patient ou de l'animal, la gravité de son état général, la tolérance au traitement, les effets secondaires constatés, le type de peau.

L'invention a aussi pour objet un extrait selon l'invention ou une composition selon l'invention pour son utilisation pour prévenir et/ou traiter les troubles ou pathologies de la peau et/ou des muqueuses (gencives, parodontes, muqueuses génitales) et/ou des phanères (cheveux et ongles) immature(s), normale(s) ou mature(s)/âgée(s), avantageusement les réactions inflammatoires, les réactions d'oxydation, les désordres liés à des attaques radicalaires liées ou non à la pollution, les troubles de la barrière ou de l'homéostasie, le vieillissement, notamment le vieillissement chronologique et/ou actinique, et/ou les agressions mécaniques ou thermiques, plus avantageusement les réactions ou pathologies inflammatoires, irritatives ou les troubles de la barrière ou de l'homéostasie de la peau, des phanères et/ou des muqueuses (gencives, parodontes, muqueuses génitales) immature(s), normale(s) ou mature(s)/âgée(s).

L'invention a aussi pour objet un extrait selon l'invention ou une composition selon l'invention pour son utilisation pour prévenir et/ou traiter les troubles vasculaires, en particulier les rougeurs et les couperoses.

L'invention a également pour objet l'utilisation d'un extrait selon l'invention ou d'une composition selon l'invention pour la fabrication d'une composition cosmétique, pharmaceutique ou dermatologique pour prévenir et/ou traiter les troubles ou pathologies de la peau et/ou des muqueuses (gencives, parodontes, muqueuses génitales) et/ou des phanères (cheveux et ongles) immature(s), normale(s) ou mature(s)/âgée(s), avantageusement les réactions inflammatoires, les réactions d'oxydation, les désordres liés à des attaques radicalaires liées ou non à la pollution, les troubles de la barrière ou de l'homéostasie, le vieillissement, notamment le vieillissement chronologique et/ou actinique, et/ou les agressions mécaniques ou thermiques, plus avantageusement les réactions ou pathologies inflammatoires, irritatives ou les troubles de la barrière ou de l'homéostasie de la peau, des phanères et/ou des muqueuses (gencives, parodontes, muqueuses génitales) immature(s), normale(s) ou mature(s)/âgée(s).

L'invention a aussi pour objet l'utilisation d'un extrait selon l'invention ou d'une composition selon l'invention pour la fabrication d'une composition pharmaceutique, cosmétique ou dermatologique pour prévenir et/ou traiter les troubles vasculaires, en particulier les rougeurs et les couperoses.

L'invention a en outre pour objet une méthode pour prévenir et/ou traiter les troubles ou pathologies de la peau et/ou des muqueuses (gencives, parodontes, muqueuses génitales) et/ou des phanères(cheveux et ongles) immature(s), normale(s) ou mature(s)/âgée(s), avantageusement les réactions inflammatoires ; les réactions d'oxydation ; les désordres liés à des attaques radicalaires liées ou non à la pollution ; les troubles de la barrière ou de l'homéostasie ; le vieillissement, notamment le vieillissement chronologique et/ou actinique ; et/ou les agressions mécaniques ou thermiques, , plus avantageusement les réactions ou pathologies inflammatoires, irritatives ou les troubles de la barrière ou de l'homéostasie de la peau, des phanères (cheveux et ongles) et/ou des muqueuses (gencives, parodontes, muqueuses génitales) immature(s), normale(s) ou mature(s)/âgée(s), comprenant l'administration, notamment l'administration topique, d'une quantité efficace d'un extrait selon l'invention ou d'une composition selon l'invention, à un sujet en ayant besoin.

L'invention a aussi pour objet une méthode pour prévenir et/ou traiter les troubles vasculaires, en particulier les rougeurs et les couperoses, comprenant l'administration, notamment l'administration topique, d'une quantité efficace d'un extrait selon l'invention ou d'une composition selon l'invention, à un sujet en ayant besoin.

En particulier, la composition ou l'extrait selon l'invention est destiné(e) à la prévention et/ou au traitement des réactions ou pathologies inflammatoires, irritatives ou des troubles de la barrière ou de l'homéostasie de la peau, des phanères (cheveux et ongles) et/ou des muqueuses (gencives, parodontes, muqueuses génitales) immature(s), normale(s) ou mature(s)/âgée(s).

Avantageusement, les réactions, troubles ou pathologies inflammatoires, irritatives ou des troubles de la barrière ou de l'homéostasie de la peau sont : l'acné, la rosacée ou érythrocouperose, les troubles vasculaires, en particulier les rougeurs et les couperoses, la dermite du siège, la dermatite atopique, l'eczéma, la dermatite de contact, la dermatite irritative, la dermatite allergique, la dermite séborrhéique (croûte de lait), la peau sensible, la peau réactive, la peau sèche (xérose), la peau déshydratée, la peau avec rougeur, l'érythème cutané, la peau âgée ou photo âgée, la peau photosensibilisée, la peau pigmentée (mélasma, pigmentation post-inflammatoire...), la peau avec vergetures, les coups de soleil, les irritations par agents chimiques, physiques (par exemple contrainte de tension pour les femmes enceintes), bactériologiques, fongiques, le vieillissement cutanée, notamment du photo vieillissement et les désordres liés à des attaques radicalaires liées à la pollution chimique ou atmosphérique, et/ou liées à l'exposition aux UV ou aux IR.

Avantageusement, les réactions, troubles ou pathologies inflammatoires, irritatives ou des troubles de la barrière ou de l'homéostasie des muqueuses sont les gingivites (gencives sensibles des nouveaux nés, problèmes d'hygiène, dus au tabagisme ou autres), et les irritations des sphères génitales mâles ou femelles externes ou internes.

Avantageusement, les réactions, troubles ou pathologies inflammatoires, irritatives ou des troubles de la barrière ou de l'homéostasie des phanères sont les ongles cassants, les ongles fragiles, les cheveux fragilisés, les cheveux cassants, les cheveux secs, l'alopécie, les dermites séborrhéiques, et les dermites folliculites.

De manière particulièrement avantageuse, la composition ou l'extrait selon l'invention est destiné(e) comme anti-âge, contre le vieillissement cutané, en particulier chronologique et actinique, par exemple lié à l'exposition solaire ou à des phénomènes d'inflammation, ou encore pour agir sur les peaux irritées.

L'invention a également pour objet l'utilisation cosmétique d'un extrait selon l'invention ou d'une composition selon l'invention dans le traitement et /ou la prévention de la peau déshydratée ; la peau avec rougeur ; la peau âgée ou photo âgée ; la peau photosensibilisée ; du vieillissement cutané, notamment du photo vieillissement ; et des désordres liés à des attaques radicalaires liées à la pollution chimique ou atmosphérique, et/ou liées à l'exposition aux UV ou aux IR.

L'invention a également pour objet l'utilisation cosmétique d'un extrait selon l'invention ou d'une composition selon l'invention pour le soin des phanères, notamment pour le traitement et/ou la prévention des ongles cassants ; des ongles fragiles ; des cheveux fragilisés ; des cheveux cassants ; et des cheveux secs.

L'invention concerne également un procédé de soin cosmétique de la peau et/ou des phanères et/ou des muqueuses, en vue d'améliorer leur état et/ou leur aspect, consistant à administrer une composition ou un des extraits selon la présente invention.

En particulier, l'invention concerne un procédé de soin cosmétique de la peau et/ou des phanères, en vue d'en prévenir les altérations de la barrière et sa déshydratation, consistant à appliquer sur la peau et/ou les phanères une composition ou un extrait selon la présente invention.

L'invention concerne un procédé de soin cosmétique de la peau et/ou des phanères, en vue d'en prévenir le vieillissement consistant à appliquer sur la peau et/ou les phanères une composition ou un extrait selon la présente invention.

L'invention concerne également l'utilisation cosmétique d'une composition ou d'un extrait selon l'invention pour hydrater la peau et/ou les muqueuses, pour agir sur l'élasticité ou la fermeté de la peau, en particulier comme agent tenseur ou anti-rides, pour agir sur les peaux sensibles, ou encore pour agir contre la pollution.

### DESCRIPTION DES FIGURES

La Figure 1 représente les forces contractiles développées par des fibroblastes de ride (FR) comparativement à des fibroblastes sains (FS).
La Figure 2 représente les forces contractiles développées par des fibroblastes sains (FS) en présence ou non de l'extrait KPO selon l'invention.
La Figure 3 représente les forces contractiles développées par des fibroblastes de ride (FR) en présence ou non de l'extrait KPO selon l'invention.

Les exemples qui suivent permettent d'illustrer l'invention.

### Exemple 1 : extraits selon l'invention

Des extraits de calices de *Bombax costatum* riche en polyphénols sont obtenus selon le procédé suivant :
a) mise en solution des calices de *Bombax costatum* broyés dans un mélange eau/propanediol 30/70, 20/80 ou 10/90 p/p
b) extraction 2h à 50°C
c) séparation solide/liquide par filtrations successives.

L'extrait liquide riche en polyphénols ainsi obtenu présente les caractéristiques suivantes (% en poids de molécules / extrait sec de l'extrait obtenu) :

| **Mélange eau/propanediol (p/p)** | **30/70** | **20/80** | **10/90** |
|---|---|---|---|
| Extrait sec (2h, 105°C, étuve ventilée) | 1.6% | 1.2% | 1.3% |
| Polyphénols totaux (éq. Acide gallique) | 19% | 23% | 22% |
| OPC (éq. Catéchine) | 20.6% | 22.5% | 24% |
| Flavonoïdes (éq. Rutine) | 4.6 % | 6% | 5% |
| Activité antioxydante (DPPH) | 31 (µg) | 12 (µg) | 27(µg) |

L'extrait de kapokier selon l'invention obtenu selon l'exemple 1 (20/80) est aussi appelé extrait KPO dans le texte et dans les Figures.

### Exemple 2: extrait hors de l'invention

Un extrait de calices de *Bombax costatum* riche en polyphénols est obtenu selon le procédé suivant :
a) mise en solution des calices de *Bombax costatum* broyés dans un mélange eau/éthanol 20/70 p/p ;
b) extraction 2h à 75°C ; et
c) séparation solide/liquide par filtrations successives.

L'extrait liquide riche en polyphénols ainsi obtenu présente les caractéristiques suivantes (% molécules /extrait sec de l'extrait obtenu) :

| | |
|---|---|
| • Extrait sec (2h, 105°C, étuve ventilée) : | 1.05% |
| • Polyphénols totaux (éq. Acide gallique) : | 24% |
| • OPC (éq. Catéchine) : | 14% |
| • Flavonoïdes (éq. Rutine) : | 7.8% |
| • Activité antioxydante (DPPH) : | 34 (µg) |

### Exemple 3 : Tests d'activités biologiques de l'extrait selon l'invention

Dans la suite de l'exemple, l'extrait testé, nommé extrait KPO, est un extrait selon l'invention obtenu selon l'exemple 1 (mélange eau/propanediol 20/80).

### 1 -Screening d'expression génique sur cultures de fibroblastes, kératinocytes et explants de peau

L'effet de l'extrait KPO sur l'expression d'ARN messagers caractéristiques des besoins cosmétiques a été évalué dans des modèles caractéristiques de la peau : des kératinocytes épidermiques humains normaux ou des fibroblastes dermiques humains normaux ont été incubés pendant 24h en présence de l'extrait KPO à 0,0005% et 0,001% (concentration en extrait sec : e.s.), par ailleurs, des explants de peau ont été incubés 24h en présence de l'extrait de KPO à 0,005% et 0,001% (e.s.). A la fin de l'incubation, les ARNm ont été extraits et l'expression de 90 gènes d'intérêt a été analysée par RT-qPCR.

Les résultats ont montré un potentiel d'activité biologique de l'extrait KPO sur les axes suivants :
- Néosynthèse, structuration et remodelage de la matrice dermique : stimulation des gènes DCN, SPARC, BGN, COL3A1, TP63, TGFB1 ;
- Adhésion et fonction barrière : stimulation des gènes CD44, CTNNA1, ITGA2, SDC1, LAMC2, TNC, COL17A1, VCAN, PXN, LAMAS, S100A7 ;
- Longévité et renouvellement : stimulation des gènes SIRT6, ATP5A1, INSR, EGF, SIRT3 ;
- Pigmentation : stimulation des gènes POMC et PMEL ;
- Détoxification et défense anti-oxydante : stimulation des gènes BSG, CAT, GLRX, MSRA, MSRB2, TXN, SOD2, PPIA, HMOX1, HSPB1, DGAT1, GBA.

En résumé, deux activités principales de l'extrait KPO ressortent de cette analyse. La première est l'adhésion et la fonction barrière avec une forte expression de gènes impliqués dans l'adhésion cellule-cellule ou cellule-matrice. Cette activité est renforcée par des acticités de néo-synthèse et de structuration de la matrice extracellulaire qui révèlent une action sur la fermeté des tissus. La réponse au stress est également très représentée avec l'induction d'un grand nombre de gènes dont certains présentent des inductions particulièrement fortes. Dans cette réponse au stress, la protection contre le stress oxydatif est prédominante. Des voies de réponse à d'autres types de stress et de voies de détoxification sont également activées.

### 2 - Expression génique de marqueurs de la matrice extra-cellulaire dermique et de communication cellulaire

L'effet de l'extrait KPO a été évalué sur l'expression génique de marqueurs impliqués dans les communications cellulaires et dans l'organisation de la matrice extra-cellulaire dermique dans des fibroblastes : des fibroblastes dermiques humains normaux ont été incubés pendant 6h ou 24h en présence de l'extrait KPO à 0,0005% ou 0,001% (e.s.) ou de TGFβ1 à 5ng/ml (contrôle positif d'induction des marqueurs de la matrice dermique). A la fin de l'incubation, l'expression génique des marqueurs suivants a été analysée par RT-qPCR :
- **CTNNA1 (Caténine α) :** se lie aux cadhérines pour favoriser les interactions cellulaires.
- **Syndecan** : protéoglycane servant à consolider les interactions cellule/cellule et cellule/matrice.
- **Laminine 5 (α3β3γ2)** : molécule d'adhésion à la membrane basale.
- **TNC (Ténacine C) :** protéines de la matrice extracellulaire qui intervient dans la cohésion de la jonction Dermo-épidermique et qui favorise la synthèse de collagène VII.
- **SPARC :** protéine associée à divers constituants de la matrice extracellulaire et qui participe à son organisation.
- **BGN (Biglycan) :** protéoglycane impliquée dans l'assemblage des fibres de collagènes.
- **Versican :** composant majeur de la MEC et joue un rôle central dans la morphogénèse et le maintien des tissus. Il participe à la formation des fibres élastiques de la peau.
- Les collagènes, qui apportent au derme ses propriétés de fermeté et de résistance à la pression, représentent environ 70% des composants de la Matrice Extra Cellulaire (MEC) dermique, avec en majorité le **collagène de type I** (85-90% du collagène dermique) qui est souvent associé au **collagène de type III.**
- **Collagène IV et VII :** glycoprotéines, constituants de la Jonction Dermo-Epidermique
- **Fibronectine :** est une glycoprotéine de haut poids moléculaires. Elle joue un rôle dans la structure de la MEC dermique.
- Les fibres élastiques sont responsables de l'élasticité de la peau. Elles résultent de l'assemblage de deux composants majeurs : **l'élastine** (1 à 3% du derme) et les micro fibrilles.
- **LOXL:** responsable de l'assemblage des fibres d'élastine.
- **Décorine :** protéoglycane impliqué dans l'assemblage de la matrice.
- **Dermatopontine :** joue un rôle dans l'assemblage des fibres de collagène ; elle est importante pour le maintien de l'architecture de la matrice extra cellulaire dermique et la flexibilité du tissu.
- **Laminine 5 (α3β3)** : molécule d'adhésion à la membrane basale (Jonction Dermo-Epidermique).

Les résultats sont exprimés en quantité relative (QR) par rapport au contrôle non traité, la significativité des résultats a été évaluée par un test statistique (Analyse de Variance à un facteur suivie d'un test de Dunnett) : *p<0,05 ; **p<0,01 ; ***p<0,001 ; ns : non significatif p>0,05.

Les résultats sont regroupés dans les tableaux 1 et 2 ci-dessous. Ils montrent un effet inducteur important de l'extrait KPO sur l'ensemble des marqueurs étudiés. Suggérant un fort potentiel d'efficacité dans la structuration de matrice dermique, de la Jonction Dermo-épidermique (JDE) et les processus de communication cellulaire. L'extrait KPO a donc une activité dans l'hydratation, l'élasticité, les échanges dermiques (JDE) et le vieillissement cutané.

**Tableau 1 : Analyse de l'expression génique après 6h de traitement :**

| Gène évalué | Niveau d'expression (Quantité Relative) | | | |
|---|---|---|---|---|
| | Contrôle négatif | Contrôle positif (TGFβ1 5 ng/ml) | KPO 0,0005% | KPO 0,001% |
| SPARC | 1,00 | 1,28 ns | 1,37 *** | 1,60 ** |
| BGN | 1,00 | 1,28 ns | 1,73 ** | 2,11 *** |
| CTNNA1 | 1,00 | 1,07 ns | 1,48 * | 1,48 *** |
| Syndecan | 1,00 | 1,85 ns | 1,41 ns | 1,73 ns |
| TNC | 1,00 | 0,94 ns | 1,41 ns | 1,73 * |
| Versican | 1,00 | 1,61 ns | 1,15 ns | 1,24 ns |
| Collagène I | 1,00 | 0,84 ns | 1,33 ns | 2,08 *** |
| Collagène III | 1,00 | 0,91 ns | 1,24 ^{∗}* | 1,96 *** |
| Collagène IV | 1,00 | 1,68 *** | 1,33 ns | 1,55 ** |
| Collagène VII | 1,00 | 1,96 **** | 0,86 ns | 1,41 * |
| Fibronectine | 1,00 | 1.09 ns | 1.22 ns | 1.60 ** |
| Elastine | 1,00 | 0,72 ns | 1.26 ns | 1.96 *** |
| LOXL | 1,00 | 1,05 ns | 1,46 ** | 1,99 ** |
| Decorine | 1,00 | n.d. | 1,29 * | 1,30 * |
| Dermatopontine | 1,00 | 1,14 ns | 1,32 * | 1,49 * |
| Laminine 5α3 | 1,00 | n.d. | 2,17 * | 1,64 ns |
| Laminine 5β3 | 1,00 | n.d. | 1,48 * | 1,61 * |

**Tableau 2 : Analyse de l'expression génique après 24h de traitement :**

| Gène évalué | Niveau d'expression (Quantité Relative) | | | |
|---|---|---|---|---|
| | Contrôle négatif | Contrôle positif (TGFβ1 5 ng/ml) | KPO 0,0005% | KPO 0,001% |
| SPARC | 1,00 | 2,00 *** | 1,65 *** | 2,12*** |
| BGN | 1,00 | 2,92 *** | 2,05 *** | 2,61 ** |
| CTNNA1 | 1,00 | 1,07 ns | 1,47 * | 1,80 ** |
| Syndecan | 1,00 | 8,08 *** | 1,98 * | 2,65 * |
| TNC | 1,00 | 1,79 * | 1,53 ns | 1,72 * |
| Versican | 1,00 | 2,87 ** | 1,45 ** | 1,06 ns |
| Collagène I | 1,00 | 2,30 * | 2,31 * | 4,91 *** |
| Collagène III | 1,00 | 1,24 ns | 1,35 ns | 3,16 *** |
| Collagène IV | 1,00 | 2,81 ** | 1,60 ** | 2,08 *** |
| Collagène VII | 1,00 | 3,07 *** | 1,20 ns | 1,78 ns |
| Fibronectine | 1,00 | 2,12 * | 1,27 ns | 1,77 *** |
| Elastine | 1,00 | 3,65 ** | 2,50 * | 6,20 *** |
| LOXL | 1,00 | 1,10 ns | 1,39 * | 2,28 *** |
| Decorine | 1,00 | n.d. | 1,19 *** | 1,58 * |
| Dermatopontine | 1,00 | 1,36 * | 1,41 * | 2,00*** |
| Laminine 5α3 | 1,00 | n.d. | 1,58 * | 1,60 * |
| Laminine 5β3 | 1,00 | n.d. | 1,35 ns | 1,68 * |

### 3 - Evaluation de l'activité anti-protéase dans des fibroblastes dermiques

L'extrait KPO a été évalué pour ses capacités à limiter la production de protéase MMP1 (Matrix MetalloProteinase-1) induite par un stress inflammatoire dans des fibroblastes dermiques.

Des fibroblastes dermiques humains normaux ont été pré-incubés pendant 24 heures en présence de l'extrait KPO à 0,0005% ou 0,001% (e.s.), les fibroblastes ont ensuite été stimulés par 100nM de PMA (Phorbol Mysristate Acetate) et incubés toute une nuit toujours en présence de l'extrait KPO. La production de MMP1 a été évaluée par dosage ELISA dans les surnageants cellulaires. Les résultats sont exprimés en pg de MMP1 par mg de protéines totales (déterminées par BC Assay). La significativité a été évaluée au moyen d'une analyse de variance à un facteur suivie d'un test de Tukey : *p<0,05 ; ***p<0,001 ; ns : non significatif p>0,05.

Les résultats sont regroupés dans le tableau 3 ci-dessous. L'extrait KPO a significativement inhibé le relargage de MMP1 induit par un stress au PMA dans des fibroblastes ; ce résultat montre un effet protecteur de la matrice dermique et montre ainsi un effet anti-âge chronologique et actinique.

**Tableau 3 : Production de MMP1 dans des fibroblastes stimulés par le PMA:**

| | MMP1 (pg/mg de protéines) | |
|---|---|---|
| Contrôle | 3,859 ± 0,623 | |
| PMA | 11,358 ± 2,560 | +194% vs contrôle *** |
| KPO à 0,0005% | 8,230 ± 0,768 | -28% vs PMA (ns) |
| KPO à 0,001% | 7,727 ± 0,770 | -32% vs PMA * |

### 4 - Evaluation du potentiel anti-inflammatoire

L'activité anti-inflammatoire de l'extrait KPO a été évaluée dans des kératinocytes stimulés par un traitement au PMA.

Des kératinocytes épidermiques humains normaux ont été pré-incubés pendant 24 heures en présence de l'extrait KPO à 0,001%, 0,002% ou 0,005% (e.s) ou des molécules anti-inflammatoires de référence dexaméthasone à 0,1µM ou indométhacine à 0,1µM. Les kératinocytes ont ensuite été stimulés par 10µg/ml de PMA (Phorbol Mysristate Acetate) et incubés overnight toujours en présence de l'extrait KPO ou des molécules de référence. La production des médiateurs inflammatoires Interleukine-8 (IL8), Interleukine-1β (IL1β) et Prostaglandine E2 (PGE2) a été évaluée par dosages ELISA dans les surnageants cellulaires. La significativité a été évaluée au moyen d'une analyse de variance à un facteur suivie d'un test de Tukey : ^{∗}p<0,05 ; ^{∗∗}p<0,01 ; ^{∗∗∗}p<0,001 ; ns : non significatif p>0,05.

Les résultats sont regroupés dans les tableaux 4, 5 et 6 suivants. L'extrait KPO a significativement inhibé le relargage des cytokines IL1β et IL8, et dans une moindre mesure PGE2, induit par un stress pro-inflammatoire dans des kératinocytes ; ces résultats montrent le potentiel d'action anti-inflammatoire de l'extrait KPO et un effet anti-âge, sur les peux sensibles, ou encore sur les peaux irritées.

**Tableau 4 : Production d'IL8 dans des kératinocytes stimulés par le PMA :**

| | IL8 (pg/ml)/ DO rouge neutre | | |
|---|---|---|---|
| | Moyenne | Ecart-type | |
| Contrôle | 47.215 | 4.711 | |
| PMA | 1420.626 | 92.557 | +2909% vs contrôle *** |
| KPO à 0,001% | 1198.008 | 154.124 | -16% vs PMA * |
| KPO à 0,002% | 795.287 | 84.255 | -44% vs PMA *** |
| KPO à 0,005% | 338.854 | 18.731 | -76% vs PMA *** |

**Tableau 5 : Production d'IL1β dans des kératinocytes stimulés par le PMA :**

| | ILlBeta (pg/ml)/ DO rouge neutre | | |
|---|---|---|---|
| | Moyenne | Ecart-type | |
| Contrôle | 0.007 | 0.000 | |
| PMA | 0.040 | 0.006 | +451 % vs contrôle *** |
| Dexa + PMA | 0.026 | 0.002 | -35% vs PMA ** |
| KPO à 0,001% | 0.018 | 0.003 | -55% vs PMA *** |
| KPO à 0,002% | 0.017 | 0.003 | -57% vs PMA *** |
| KPO à 0,005% | 0.017 | 0.003 | -57% vs PMA *** |

**Tableau 6 : Production de PGE2 dans des kératinocytes stimulés par le PMA :**

| | PGE2 (pg/ml)/ DO rouge neutre | | |
|---|---|---|---|
| | Moyenne | Ecart-type | |
| Contrôle | 220.878 | 18.449 | |
| PMA | 802.843 | 75.214 | +263% vs contrôle *** |
| Dexa + PMA | 617.645 | 61.477 | -23% vs PMA ** |
| Indo + PMA | 181.780 | 15.506 | -77% vs PMA *** |
| KPO à 0,001% | 631.360 | 24.516 | -21% vs PMA * |
| KPO à 0,002% | 701.909 | 60.353 | -13% vs PMA (ns) |
| KPO à 0,005% | 735.274 | 60.135 | -8% vs PMA (ns) |

### 5 - Evaluation du potentiel anti-oxydant

L'efficacité anti-oxydante de l'extrait KPO a été évaluée dans un modèle de dosage des ROS (Reactive Oxygen Species ou Espèces Réactives de l'Oxygène) intracellulaire induits par un stress pro-oxydant H₂O₂.

Des kératinocytes épidermiques humains normaux ont été pré-incubés pendant 24 heures en présence de l'extrait KPO à 0,001% ; 0,002% ou 0,005% (e.s.) ou des molécules de référence anti-oxydantes Quercétine à 10µM ou Vitamine C à 500µM. Les tapis cellulaires ont ensuite été mis en présence de la sonde DCFH-DA avant induction du stress oxydatif par 100µM d'H₂O₂. La quantité de ROS produits a été évaluée par mesure de la fluorescence émise lorsque la sonde est oxydée. Les résultats ont été analysés statistiquement par une analyse de variance à un facteur suivie d'un test de Tukey : ^{∗∗}p<0,01 ; ^{∗∗∗}p<0,001.

Les résultats sont regroupés dans le tableau 7 suivant. L'extrait KPO a significativement inhibé la production de ROS induits par l'H₂O₂. Ces résultats montrent un potentiel anti-oxydant puissant. L'extrait KPO peut ainsi être avantageusement utilisé contre la pollution et le vieillissement.

**Tableau 7 : Production de ROS dans des kératinocytes soumis à un stress H₂O₂ :**

| | ROS (Unité Arbitraire) | | |
|---|---|---|---|
| | Moyenne | Ecart-type | |
| Contrôle | 18564.862 | 12917.522 | |
| H₂O₂ à 100µM | 133342.523 | 52149.942 | +618% vs contrôle *** |
| Quercétine à 10µmol/l | 12959.361 | 2587.250 | -90% vs H₂O₂ *** |
| Vit C 500µM | 12807.703 | 4220.635 | -90% vs H₂O₂ *** |
| KPO à 0,001% | 68750.713 | 10417.179 | -48% vs H₂O₂ ** |
| KPO à 0,002% | 59761.384 | 15339.601 | -55% vs H₂O₂ *** |
| KPO à 0,005% | 26779.475 | 6550.110 | -80% vs H₂O₂ *** |

### 6 - Evaluation in vitro de l'effet anti-ride et de l'effet tenseur

L'effet « tenseur » de l'extrait KPO a été évalué dans le système GlasBox^{®} sur des fibroblastes de fond de ride et sur des fibroblastes de peau non ridée avoisinante (fibroblastes « sains »). Le système GlaSbox^{®} permet de mesurer, au sein d'un derme équivalent, les forces contractiles développées par les fibroblastes. Les fibroblastes issus de fond de ride développent moins de forces de contraction que ceux de la peau non ridée avoisinante, il est ainsi possible d'évaluer l'effet « anti-ride » d'un composé.

Des dermes équivalents ont été préparés en mélangeant les fibroblastes (de peau saine ou de ride) à une solution de collagène. Les fibroblastes provenaient d'un prélèvement obtenu chez un même patient (femme de 65 ans) au fond de la ride, d'une part, et de la peau non ridée avoisinante, d'autre part. Ce mélange a été coulé dans les cuves rectangulaires de la GlasBox^{®}. Un milieu contenant ou non l'extrait KPO à 0,0005% ou 0,001% (e.s.) a été ajouté. Dans chacune de ces cuves, plongent deux lames flexibles en silicium. Le derme équivalent se développe ainsi entre deux lames équipées d'un système de jauge de contrainte (« capteur »). Sous l'influence de la force de rétraction développée par les fibroblastes, les lames de silicium se déforment ; cela se traduit par une variation de la valeur de la résistance électrique de la jauge de contrainte. Cette variation, mesurée en temps réel, est représentative de la force développée au sein du derme équivalent. Les forces isométriques ont ainsi été mesurées pendant 24 heures.

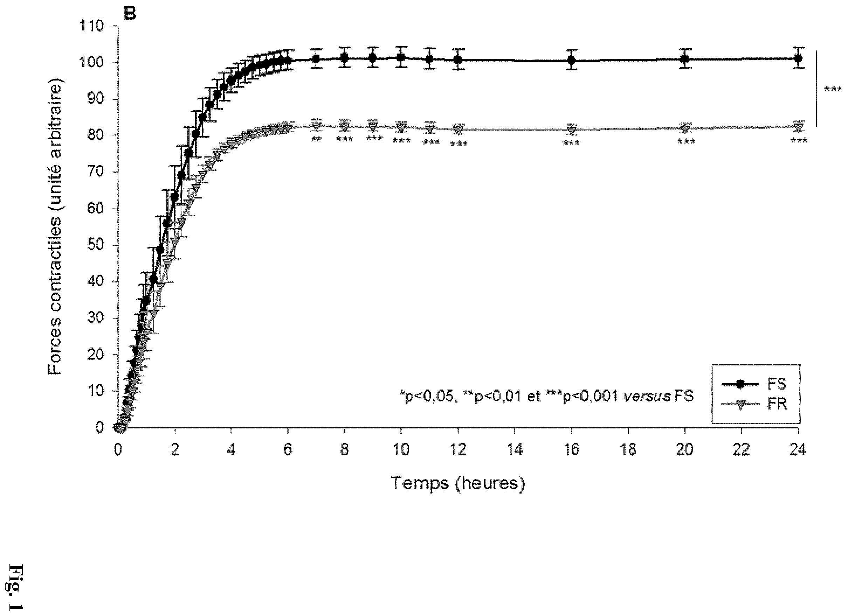

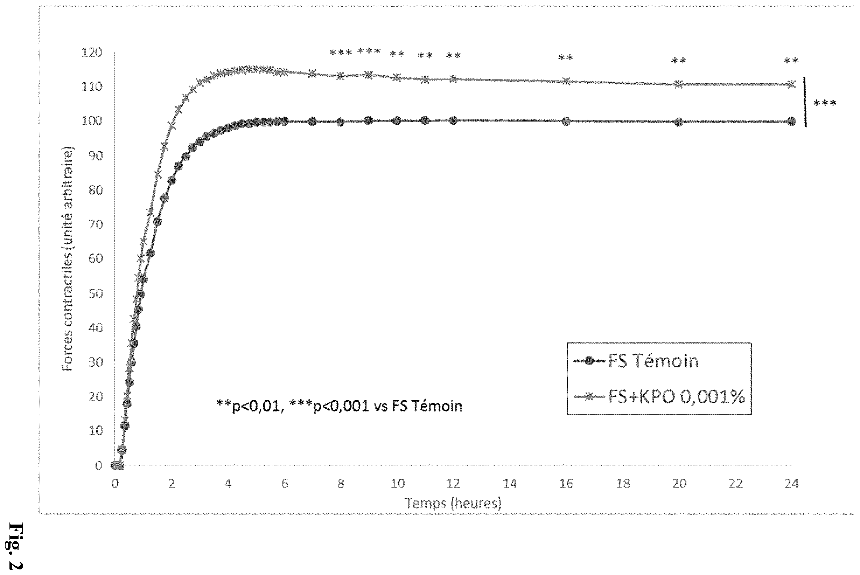

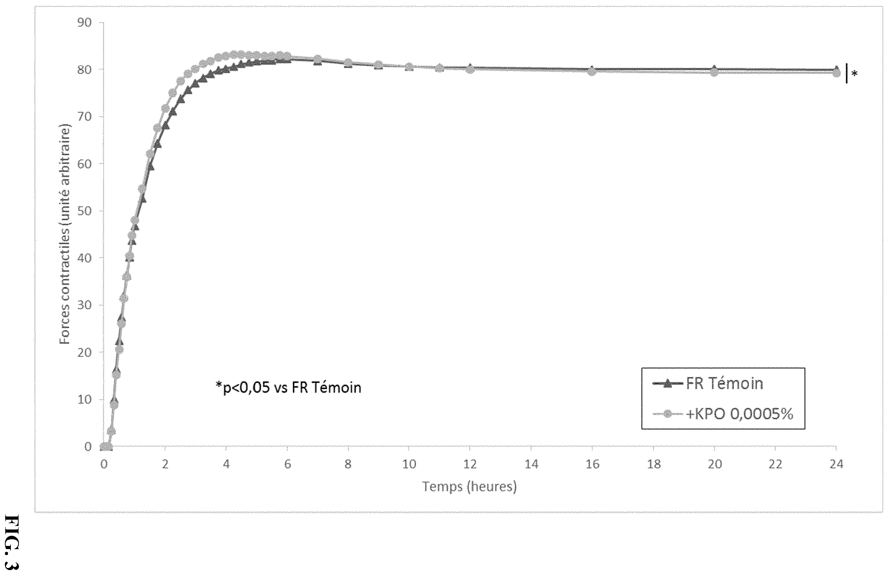

Les résultats sont regroupés dans les Figures 1 à 3 et le tableau 8. Comme attendu, les forces contractiles développées par les fibroblastes de ride (FR) sont significativement diminuées par rapport à celles développées par les fibroblastes sains (FS).

Dans les fibroblastes de ride (FR), l'extrait KPO à 0,0005% a significativement augmenté les forces contractiles. Ce résultat montre un effet anti-ride.

Appliqué sur des fibroblastes sains, l'extrait KPO a significativement augmenté les forces contractiles. L'analyse des courbes Glasbox^{®} montre que l'AUC (aire sous la courbe) et le maximum de contraction sont significativement augmentés en présence de l'extrait. Ces résultats montrent un effet tenseur de l'extrait.

**Tableau 8 : Analyse des courbes de Glasbox (FS)**

| | FS Témoin | | FS + 0,001% de KPO | |
|---|---|---|---|---|
| Paramètres calculés | Moyenne | sem | Moyenne | sem |
| AUC | 2278,6 | 40,2 | 2574,5** | 101,1 |
| Max | 97,79 | 1,46 | 112,44** | 4,32 |

| | | | | |
|---|---|---|---|---|
| ^{∗∗}*p<0,01 vs FS Témoin* - *Analyse de variance à un facteur suivie d'un test de Fisher* | | | | |

### 7- Evaluation de l'effet de l'extrait KPO sur l'activité mitochondriale dans des fibroblastes de peau

Des fibroblastes dermiques humains normaux ont été incubés pendant 24 heures en présence de l'extrait KPO à 0,001% (e.s.). A l'issue du traitement, les cellules ont été récoltées par trypsination et incubées en présence d'une sonde fluorescente sensible à l'oxygène afin de mesurer la consommation d'oxygène en temps réel au spectrofluorimètre.

Les résultats sont regroupés dans le tableau 9. Ces résultats montrent que l'extrait KPO a induit une augmentation de la respiration globale. Ainsi, l'extrait KPO induit une stimulation de l'activité de la chaine respiratoire, effet représentatif d'une stimulation des fonctions mitochondriales.

**Tableau 9 : Mesure de la consommation d'oxygène dans des NHDF**

| | Consommation d'O2 (%) |
|---|---|
| Contrôle non traité | 100 |
| KPO à 0,001% | 145,40 |

## Revendications

1. Extrait riche en polyphénols de fleurs de *Bombax costatum,* de préférence de calices de *Bombax costatum,* comprenant au moins 15% en poids de polyphénols, par rapport au poids total de l'extrait sec, et au moins 15 % en poids d'oligo-proanthocyanidines (OPC), exprimé en équivalent catéchine, par rapport au poids total de l'extrait sec.

2. Extrait selon la revendication 1, comprenant au moins 20%, en poids de polyphénols, par rapport au poids total de l'extrait sec.

3. Extrait selon l'une quelconque des revendications 1 et 2, dans lequel ledit extrait comprend en outre au moins 6% en poids d'acides organiques, en particulier d'alpha-hydroxy acides, tels que les acides acétique, malique, lactique et citrique ou leurs mélanges, par rapport au poids de l'extrait sec.

4. Extrait selon l'une quelconque des revendications 1 à 3, dans lequel ledit extrait comprend en outre au moins 5 % en poids de flavonoïdes, exprimé en équivalent rutine, par rapport au poids total de l'extrait sec.

5. Extrait selon l'une quelconque des revendications 1 à 4 **caractérisé en ce qu'**il est obtenu par extraction solide/liquide des fleurs, et particulièrement des calices, de *Bombax costatum,* dans un solvant choisi parmi les mélanges binaires eau/glycol.

6. Extrait selon l'une quelconque des revendications 1 à 5, **caractérisé en ce qu'**il est obtenu par extraction solide/liquide des fleurs, et particulièrement des calices, de *Bombax costatum,* dans un solvant choisi parmi les mélanges binaires eau/glycol, dans une proportion comprise entre 30 et 90% de glycol dans l'eau.

7. Procédé de préparation d'un extrait riche en polyphénols de fleurs, de préférence de calices, de *Bombax costatum,* tel que défini selon l'une quelconque des revendications 1 à 4, comprenant au moins une étape d'extraction solide/liquide dans un solvant choisi parmi les mélanges binaires eau/glycol.

8. Procédé selon la revendication 7, **caractérisé en ce qu'**il comprend les étapes successives suivantes :
a) broyage des fleurs, en particulier des calices ;
b) extraction solide/liquide des fleurs, en particulier des calices, broyées dans un solvant choisi parmi les mélanges binaires eau/glycol;
c) séparation de la phase solide et de la phase liquide par décantation, et/ou centrifugation et/ou filtrations successives ; et
d) éventuellement séchage de l'extrait obtenu à l'étape c).

9. Procédé selon la revendication 7 ou 8, **caractérisé en ce que** ledit solvant d'extraction solide/liquide est choisi parmi les mélanges binaires eau/glycol,dans une proportion comprise entre 30 et 90% de glycol dans l'eau.

10. Procédé selon l'une quelconque des revendications 7 à 9, **caractérisé en ce que** le glycol est un glycol végétal, en particulier un propanediol ou le propylène glycol, notamment le 1,3-propanediol.

11. Composition comprenant en tant que principe actif un extrait riche en polyphénols de fleurs, de préférence de calices, de *Bombax costatum,* tel que défini dans l'une quelconque des revendications 1 à 6 ou d'un extrait susceptible d'être obtenu par le procédé selon l'une quelconque des revendications 7 à 10, et avantageusement un excipient approprié.

12. Extrait selon l'une quelconque des revendications 1 à 6 ou extrait susceptible d'être obtenu par le procédé selon l'une quelconque des revendications 7 à 10 ou composition selon la revendication 11, pour son utilisation pour prévenir et/ou traiter :
- des troubles ou pathologies de la peau et/ou des muqueuses et/ou des phanères, avantageusement des réactions inflammatoires, des réactions d'oxydation, des désordres liés à des attaques radicalaires liées ou non à la pollution, des troubles de la barrière ou de l'homéostasie, des agressions mécaniques et/ou thermiques, et/ou
- des troubles vasculaires, notamment les rougeurs ou les couperoses, et/ou
- les désordres liés à des attaques radicalaires liées à l'exposition aux UV et aux IR ; et/ou
- la peau photosensibilisée.

13. Procédé de soin cosmétique de la peau et/ou des phanères et/ou des muqueuses, en vue d'améliorer leur état et/ou leur aspect, consistant à administrer un extrait tel que défini selon l'une quelconque des revendications 1 à 6 ou un extrait susceptible d'être obtenu par le procédé selon l'une quelconque des revendications 7 à 10 ou une composition telle que définie selon la revendication 11.

14. Procédé de soin cosmétique selon la revendication 13, **caractérisé en ce qu'**il est destiné à prévenir les altérations de la barrière de la peau et sa déshydratation et/ou à prévenir le vieillissement cutané.

15. Utilisation cosmétique d'un extrait selon l'une quelconque des revendications 1 à 6 ou d'un extrait susceptible d'être obtenu par le procédé selon l'une quelconque des revendications 7 à 10 ou d'une composition selon la revendication 11 dans le traitement et /ou la prévention de la peau déshydratée ; la peau avec rougeur ; la peau âgée ou photo âgée ; le vieillissement cutané, notamment le photo vieillissement ; et les désordres liés à des attaques radicalaires liées à la pollution chimique ou atmosphérique.

## Patentansprüche

1. Extrakt, der reich an Polyphenolen von Blüten von *Bombax costatum,* vorzugsweise von Blütenkelchen von *Bombax costatum* ist, umfassend mindestens 15 Gew.-% Polyphenole im Verhältnis zum Gesamtgewicht des Trockenextrakts und mindestens 15 Gew.-% Oligoproanthocyanidine (OPC), ausgedrückt in Catechinäquivalent, im Verhältnis zum Gesamtgewicht des Trockenextrakts.

2. Extrakt nach Anspruch 1, umfassen mindestens 20 Gew.-% Polyphenole im Verhältnis zum Gesamtgewicht des Trockenextrakts.

3. Extrakt nach einem der Ansprüche 1 und 2, wobei der Extrakt ferner mindestens 6 Gew.-% organische Säuren, vor allem Alphahydroxysäuren wie Essig-, Apfel-, Milch- und Citronensäure oder deren Gemische im Verhältnis zum Gewicht des Trockenextrakts umfasst.

4. Extrakt nach einem der Ansprüche 1 bis 3, wobei der Extrakt ferner mindestens 5 Gew.-% Flavonoide, ausgedrückt in Rutinäquivalent, im Verhältnis zum Gesamtgewicht des Trockenextrakts umfasst.

5. Extrakt nach einem der Ansprüche 1 bis 4, **dadurch gekennzeichnet, dass** er durch Fest-Flüssig-Extraktion der Blüten und insbesondere der Blütenkelche von *Bombax costatum* in einem Lösungsmittel gewonnen wird, das aus den binären Wasser-Glycol-Gemischen ausgewählt ist.

6. Extrakt nach einem der Ansprüche 1 bis 5, **dadurch gekennzeichnet, dass** er durch Fest-Flüssig-Extraktion der Blüten und insbesondere der Blütenkelche von *Bombax costatum* in einem Lösungsmittel gewonnen wird, das aus den binären Wasser-Glycol-Gemischen in einem Verhältnis gewonnen wird, das zwischen 30 und 90 % Glycol in Wasser liegt.

7. Verfahren zur Herstellung eines Extrakts, der reich an Polyphenolen von Blüten, vorzugsweise von Blütenkelchen, von *Bombax costatum* nach einem der Ansprüche 1 bis 4 ist, umfassend mindestens einen Schritt der Fest-Flüssig-Extraktion in einem Lösungsmittel, das aus den binären Wasser-Glycol-Gemischen ausgewählt ist.

8. Verfahren nach Anspruch 7, **dadurch gekennzeichnet, dass** es die folgenden aufeinanderfolgenden Schritte umfasst:
a) Zerkleinern der Blüten, vor allem der Blütenkelche;
b) Fest-Flüssig-Extraktion der in einem zerkleinerten Blüten, vor allem der Blütenkelche, in einem Lösungsmittel, das aus den binären Wasser-Glycol-Gemischen ausgewählt ist;
c) Separieren der festen Phase und der flüssigen Phase durch Dekantieren und/oder Zentrifugieren und/oder aufeinanderfolgende Filtrationen; und
d) eventuell Trocknen des in Schritt c) erhaltenen Extrakts.

9. Verfahren nach Anspruch 7 oder 8, **dadurch gekennzeichnet, dass** das Fest-Flüssig-Extraktions-Lösungsmittel aus den binären Wasser-Glycol-Gemischen in einem Verhältnis ausgewählt ist, das zwischen 30 und 90 % Glycol in Wasser liegt.

10. Verfahren nach einem der Ansprüche 7 bis 9, **dadurch gekennzeichnet, dass** das Glycol ein pflanzliches Glycol, vor allem ein Propandiol oder Propylenglycol, insbesondere 1,3-Propandiol, ist.

11. Zusammensetzung, die als Wirkstoff einen Extrakt, der reich an Polyphenolen von Blüten, vorzugsweise von Blütenkelchen von *Bombax costatum* nach einem der Ansprüche 1 bis 6 oder einen Extrakt, der durch das Verfahren nach einem der Ansprüche 7 bis 10 gewinnbar ist und in vorteilhafter Weise einen geeigneten Hilfsstoff umfasst.

12. Extrakt nach einem der Ansprüche 1 bis 6 oder Extrakt, der durch das Verfahren nach einem der Ansprüche 7 bis 10 gewinnbar ist oder Zusammensetzung nach Anspruch 11 zwecks Verwendung zur Vorbeugung und/oder Behandlung:
- von Beschwerden oder Krankheiten der Haut und/oder der Schleimhäute und/oder der Hautanhangsgebilde, in vorteilhafter Weise von entzündlichen Reaktionen, Oxidationsreaktionen, Störungen, die mit Angriffen durch Radikale verbunden sind, die mit Schadstoffen verbunden sind oder nicht, Störungen der Barriere oder der Homöostase, mechanischen und/oder thermischen Aggressionen, und/oder
- vaskulären Beschwerden, vor allem Rötungen oder Couperosen, und/oder
- Störungen, die mit Angriffen durch Radikale verbunden sind, die mit der UV- oder IR-Exposition verbunden sind; und/oder
- der lichtsensibilisierten Haut.

13. Kosmetisches Pflegeverfahren der Haut und/oder der Hautanhangsgebilde und/oder der Schleimhäute zwecks Verbesserung ihres Zustands und/oder ihres Aussehens, das darin besteht, einen Extrakt nach einem der Ansprüche 1 bis 6 oder einen Extrakt, der durch das Verfahren nach einem der Ansprüche 7 bis 10 gewinnbar ist oder eine Zusammensetzung nach Anspruch 11 zu verabreichen.

14. Kosmetisches Pflegeverfahren nach Anspruch 13, **dadurch gekennzeichnet, dass** es bestimmt ist, Beeinträchtigungen der Hautbarriere und ihrer Austrocknung vorzubeugen und/oder der Hautalterung vorzubeugen.

15. Kosmetische Verwendung eines Extrakts nach einem der Ansprüche 1 bis 6 oder eines Extrakts, der durch das Verfahren nach einem der Ansprüche 7 bis 10 gewinnbar ist oder einer Zusammensetzung nach Anspruch 11 zwecks Behandlung und/oder Vorbeugung der ausgetrockneten Haut; der geröteten Haut; der alten oder lichtgealterten Haut; der Hautalterung, insbesondere der Lichtalterung; und der Störungen, die mit Angriffen durch Radikale verbunden sind, die mit chemischen oder atmosphärischen Schadstoffen verbunden sind.

## Claims

1. Polyphenol-rich extract of the flowers of *Bombax costatum,* preferably calyxes of *Bombax costatum,* comprising at least 15% by weight of polyphenols, relative to the total weight of the dry extract, and at least 15% by weight of oligoproanthocyanidins (OPC), expressed as catechin equivalent, relative to the total weight of the dry extract.

2. Extract according to claim 1 comprising at least 20% by weight of polyphenols, relative to the total weight of the dry extract.

3. Extract according to any one of claims 1 and 2, wherein said extract further comprises at least 6% by weight of organic acids, in particular alpha-hydroxy acids, such as acetic acid, malic acid, lactic acid, citric acid or mixtures thereof, relative to the weight of the dry extract.

4. Extract according to any one of claims 1 to 3, wherein said extract further comprises at least 5% by weight of flavonoids, expressed as rutin equivalent, relative to the total weight of the dry extract.

5. Extract according to any one of claims 1 to 4 **characterized in that** it is obtained by solid/liquid extraction of the flowers, and particularly the calyxes, of *Bombax costatum,* in a solvent chosen from amongst binary mixtures of water/glycol.

6. Extract according to any one of claims 1 to 5 **characterized in that** it is obtained by solid/liquid extraction of the flowers, and particularly the calyxes, of *Bombax costatum,* in a solvent chosen from amongst binary mixtures of water/glycol, in a proportion comprised between 30 and 90% of glycol in water.

7. Method for the preparation of a polyphenol-rich extract of the flowers, preferably the calyxes, of *Bombax costatum,* as defined in any one of claims 1 to 4, comprising at least one solid/liquid extraction step in a solvent chosen from amongst binary mixtures of water/glycol.

8. Method according to claim 7, **characterized in that** it comprises the following successive steps:
a) grinding of the flowers, in particular the calyxes;
b) solid/liquid extraction of the flowers, in particular the calyxes, ground in a solvent chosen from amongst binary mixtures of water/glycol;
c) separation of the solid phase and the liquid phase by decantation and/or centrifugation and/or successive filtration; and
d) optionally, drying of the extract obtained in step c).

9. Method according to claim 7 or 8, **characterized in that** the solid/liquid extraction solvent is chosen from amongst binary mixtures of water/glycol, in a proportion comprised between 30 and 90% of glycol in water.

10. Method according to any one of claims 7 to 9, **characterized in that** the glycol is a plant glycol, in particular propanediol or propylene glycol, in particular 1,3-propanediol.

11. Composition comprising as an active ingredient a polyphenol-rich extract of flowers, preferably calyxes, of *Bombax costatum,* as defined in any one of claims 1 to 6 or an extract that can be obtained from the method according to any one of claims 7 to 10, and advantageously an appropriate excipient.

12. Extract according to any one of claims 1 to 6 or an extract that can be obtained from the method according to any one of claims 7 to 10 or composition according to claim 11, for its use in the prevention and/or treatment of:
- disorders or diseases of the skin and/or mucous membranes and/or skin appendages, advantageously inflammatory reactions, oxidations reactions, disorders related to radical attacks whether linked to pollution or not, barrier or homeostasis disorders, mechanical and/or thermal aggressions, and/or
- vascular disorders, in particular redness or blotchiness, and/or
- disorders related to radical attacks linked to exposure to UV or IR; and/or
- photosensitive skin.

13. Cosmetic care method for the skin and/or skin appendages and/or mucous membranes, in view of improving their condition and/or appearance, consisting of administering an extract as defined in any one of claims 1 to 6 or an extract that can be obtained from the method according to any one of claims 7 to 10 or a composition according to claim 11.

14. Cosmetic care method according to claim 13, **characterized in that** it is intended to prevent changes in the skin barrier and its dehydration and/or to prevent cutaneous aging.

15. Cosmetic use of an extract according to any one of claims 1 to 6 or an extract that can be obtained from the method according to any one of claims 7 to 10 or a composition according to claim 11 in the treatment and/or prevention of dehydrated skin; skin with redness; elderly or photo-aged skin; cutaneous aging, in particular photo-aging; and disorders related to radical attacks linked to chemical or atmospheric pollution.
